# EUROPÄISCHE PATENTSCHRIFT

(11) **EP 2 446 921 B1**
(45) Veröffentlichungstag und Bekanntmachung des Hinweises auf die Patenterteilung: **09.05.2018**
(21) Anmeldenummer: 11187000.2
(22) Anmeldetag: 27.10.2011
(51) Int. Cl.: A61N 1/05, A61N 1/36

(54) **Auswahlschaltung für eine Elektrodenanordnung sowie Verfahren zum Betrieb einer Elektrodenanordnung**
Selection circuit for an electrode assembly and method for operating an electrode assembly
Circuit de sélection pour un agencement d'électrodes et procédé de fonctionnement d'un agencement d'électrodes

(30) Priorität: 27.10.2010 DE 102010043029
(43) Veröffentlichungstag der Anmeldung: 02.05.2012
(73) Patentinhaber: CorTec GmbH, 79110 Freiburg (DE)
(72) Erfinder: Stieglitz, Thomas, 79110 Freiburg (DE); Schüttler, Martin, 79102 Freiburg (DE); Henle, Christian, 79100 Freiburg (DE); Rickert, Jörn, 79106 Freiburg (DE)
(74) Vertreter: 2s-ip Schramm Schneider Bertagnoll Patent- und Rechtsanwälte Part mbB

(56) Entgegenhaltungen:
- US-A- 4 628 934
- US-A1- 2005 137 670
- US-B1- 6 473 653

## Beschreibung

### Gebiet der Erfindung

Die Erfindung betrifft eine Schaltung zur Auswahl einer Anzahl von Elektroden einer implantierbaren Elektrodenanordnung, ein Verfahren zum Betrieb einer implantierbaren Elektrodenanordnung mit einer erfindungsgemäßen Schaltung zur Auswahl einer Anzahl von Elektroden der Elektrodenanordnung, sowie ein Verfahren zum Herstellen einer Elektrodenanordnung mit einer Vielzahl von Elektroden.

### Hintergrund der Erfindung und Stand der Technik

In der neurologischen Diagnostik und in der Neuroprothetik werden mittels elektrischer Stimulation von Nervengewebe Krankheiten therapiert bzw. körpereigene Nervensignale zur Steuerung technischer Prothesen und Hilfsmittel eingesetzt. Hierzu werden Elektrodenanordnungen mit einer Anzahl von Elektroden, sogenannte Elektrodenarrays, verwendet, über welche die Nerven elektrisch stimuliert werden können bzw. über welche körpereigene Nervensignale abgeleitet werden können. Bei der Neuroprothetik ist es notwendig, die Elektrodenanordnung bzw. das Elektrodenarray als implantierbares System auszulegen, um eine Implantation der Elektrodenanordnung in das Gehirn oder in oder um einen peripheren Nerven eines Patienten zu ermöglichen. Die mit Hilfe einer implantierten Elektrodenanordnung abgeleiteten Gehirnströme können etwa einer Gehirn-Computer-Schnittstelle (Brain-Computer-Interface, BCI) zugeführt und dort ausgewertet werden. Mit dem Ergebnis der Auswertung kann etwa eine Prothese gesteuert werden.

Es ist bekannt, Elektrodenarrays passiv auszulegen, d.h. jede Elektrode des Elektrodenarrays mit einem elektrisch leitfähigen Kabel zu verbinden. In der prächirurgischen Epilepsie-Diagnostik werden die Elektrodenarrays mit perkutanen Kabeln in das Gehirn eines Patienten implantiert. Die Anzahl der perkutanen Kabel, welche jeweils mit einer Elektrode des Elektrodenarrays verbunden sind, begrenzt dabei die Anzahl der Elektroden des Elektrodenarrays. Die perkutanen Kabel können über Verlängerungskabel an ein Ableitsystem oder an einen Pulsgenerator angeschlossen werden. Die Anzahl der perkutanen Kabel ist aus medizinischen Indikationen allerdings begrenzt.

Hochkanalige Ableitsysteme zum Anschluss an die perkutanen Kabel der Elektrodenarrays sind meist auf eine bestimmte Anzahl von Aufnahmekanälen begrenzt, so dass nur eine bestimmte Anzahl von Elektroden des Elektrodenarrays über die perkutanen Kabel mit dem Ableitsystem gekoppelt werden können.

Des Weiteren ist bekannt, die perkutanen Kabel über einen Multiplexer mit einem Ableitsystem zu koppeln. Damit ist es möglich, verschiedene perkutane Kabel auf das Ableitsystem aufzuschalten. Die Anzahl der perkutanten Kabel kann damit größer sein als die Anzahl der Aufnahmekanäle des Ableitsystems. Der Multiplexer kann dabei extrakorporal an die perkutanen Kabel angeschlossen werden.

Um die Anzahl perkutaner Kabel zu verringern, ist es bekannt, den Multiplexer intrakorporal anzuordnen. Mit dem Multiplexer werden ausgewählte Elektroden mit den perkutanen Kabeln verbunden. Der Multiplexer benötigt eine Energieversorgung, um die Verbindungen herzustellen, aufrechtzuerhalten und elektrische Signale durchzuleiten. Dies ist insbesondere dann nachteilig, wenn der Multiplexer intrakorporal angeordnet ist. Für den Betrieb des Elektrodenarrays muss der Multiplexer stets mit Energie versorgt werden. Weil der Multiplexer eine Anzahl von Elektroden in einer vorbestimmten Abfolge mit einer Anzahl von Aufnahmekanälen eines Ableitsystems verbindet, ist eine gezielte Auswahl der mit dem Ableitsystem zu verbindenden Elektroden nicht möglich.
Aus der EP 1 446 189 B1 ist eine Erweiterungseinheit zur elektrischen Verbindung eines implantierbaren Pulsgenerators mit einem Elektrodenarray bekannt, welche die Ausgänge des Pulsgenerators mit den Elektroden des Elektrodenarrays verbindet, wobei die Anzahl der Ausgänge des Pulsgenerators geringer ist als die Anzahl der Elektroden. Die Zuordnung eines Ausgangs des Pulsgenerators zu einer Elektrode wird mit Hilfe eines programmierbaren Schalters vorgenommen. Die programmierbaren Schalter ermöglichen eine selektive Auswahl der durchzuschaltenden Elektrodenkanäle.

Aus der US 4,628,934 A ist eine Multielektrodenanordnung für einen Herzschrittmacher bekannt. Die Multielektrodenanordnung umfasst eine Auswahlschaltung, mit der einzelne Elektroden aktiviert oder deaktiviert werden können. Die Auswahlschaltung umfasst elektronische Schalter, mit denen einzelne Elektroden mit dem Herzschrittmacher gekoppelt werden können. Die elektrische Energie für die Auswahlschaltung kann auf induktiver Basis bereitgestellt werden. Aus der US 6,473,653 B1 ist ein implantierbares Elektrodensystem mit einer Anzahl von Elektroden bekannt. Die Elektroden sind mit einem Controller verbunden, der wiederum mit einem Mikroprozessor verbunden ist. Der Mikroprozessor umfasst einen Speicher, in dem Informationen über die zu aktivierenden Elektroden gespeichert sind. Diese Informationen werden über eine elektrische Leitung an den Controller übertragen, der dann mittels elektrischer Schalter die entsprechenden Elektroden aktiviert

### Aufgabe der Erfindung

Aufgabe der vorliegenden Erfindung ist es daher, Vorrichtung anordnung sowie ein Verfahren zum Betrieb einer Vorrichtung bereitzustellen, welche einen besonders gesundheitsschonende Handhabung erlauben, ohne dabei die aus dem Stand der Technik bekannten Nachteile aufzuweisen.

### Erfindungsgemäße Lösung

Diese Aufgabe wird durch eine Vorrichtung gemäß Anspruch 1 und ein Verfahren gemäß Anspruch 4 gelöst. Vorteilhafte Ausgestaltungen und Weiterbildungen der Erfindung sind in den jeweiligen abhängigen Ansprüchen angegeben.

### Kurzbeschreibung der Figuren

Weitere Einzelheiten und Merkmale der Erfindung sowie Ausführungsbeispiele der Erfindung ergeben sich aus der nachfolgenden Beschreibung in Verbindung mit der Zeichnung. Es zeigt:
- Fig. 1: eine Elektrodenanordnung mit einer Anzahl von Elektroden, wobei die Elektroden über elektrische Leitungen mit einem Stecker verbunden sind;
- Fig. 2: eine erfindungsgemäße Schaltung zur Auswahl einer Anzahl von Elektroden einer implantierbaren Elektrodenanordnung, wobei die Schaltung in einem Stecker angeordnet ist, welcher mit den Elektroden eines Elektrodenarrays gekoppelt ist;
- Fig. 3: eine Elektrodenanordnung mit einer Anzahl von Elektroden, wobei die Erfindungsgemäße Schaltung zur Auswahl einer Anzahl von Elektroden auf dem Elektrodenträger angeordnet ist; und
- Fig. 4: eine Ausgestaltung einer erfindungsgemäßen Schaltung zur Auswahl einer Anzahl von Elektroden einer implantierbaren Elektrodenanordnung.

### Detaillierte Beschreibung der Erfindung

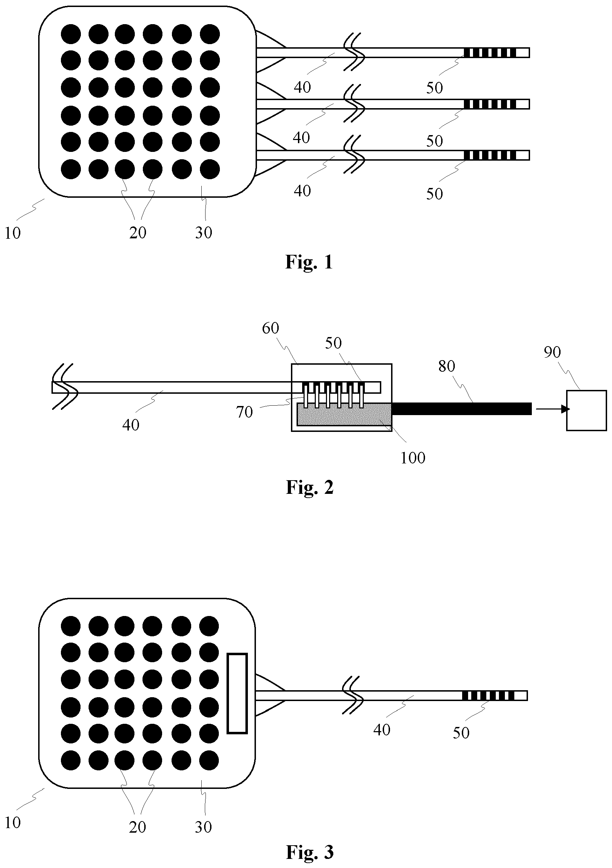

**Fig. 1** zeigt eine implantierbare Elektrodenanordnung 10 mit einer Anzahl von Elektroden 20. Die Elektroden 20 sind auf ein Substrat 30 aufgebracht bzw. in ein Substrat 30 integriert. Über elektrisch leitfähige Drähte sind die Elektroden 20 jeweils in Kabeln 40 mit Anschlusskontakten 50 verbunden. Die Anschlusskontakte 50 können an einen Stecker angeschlossen sein. In der in Fig. 1 gezeigten Ausführungsform der implantierbaren Elektrodenanordnung 10 sind drei Kabel 40 vorgesehen, über welche jeweils eine Anzahl von Elektroden 20 mit elektrisch leitfähigen Drähten mit den jeweiligen Anschlusskontakten 50 verbunden sind.

Die erfindungsgemäße Schaltung zur Auswahl einer Anzahl von Elektroden 20 einer implantierbaren Elektrodenanordnung 10 kann entweder in einer Steckverbindung oder direkt auf dem Substrat 30 angeordnet sein, auf welchem auch die Elektroden 20 angeordnet sind.

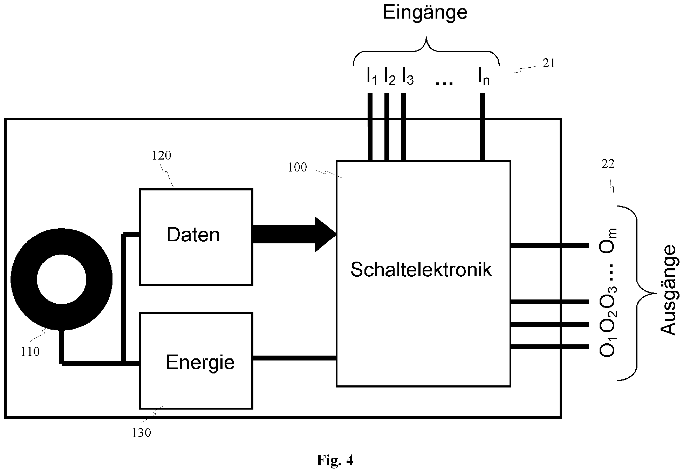

Eine Anordnung der erfindungsgemäßen Schaltung in einer Steckverbindung ist in **Fig. 2** gezeigt. Die Steckverbindung 60 weist Anschlüsse 70 auf, an welchen die Elektroden 20 bzw. die zu den Elektroden 20 führenden Drähte an die Steckverbindung 60 angeschlossen werden. Die Anschlüsse 70 der Steckverbindung 60 sind mit Eingängen (hier nicht gezeigt) der Signalschnittstelle der Auswahlschaltung gekoppelt. Die Ausgänge der Signalschnittstelle sind über elektrisch leitfähige Drähte mit einer Ableitungseinrichtung und/oder einer Stimulationseinrichtung 90 gekoppelt. Erfindungsgemäß ist die Anzahl der Eingänge der Signalschnittstelle größer als die Anzahl der Ausgänge der Signalschnittstelle. Mit Hilfe einer Schaltelektronik 100, welche mit Bezug auf Fig. 4 näher beschrieben wird, werden die Ausgänge der Signalschnittstelle mit Eingängen der Signalschnittstelle verbunden, wobei das Verbinden mit einer Anzahl von programmierbaren Schaltern bewerkstelligt wird.

Die Steckverbindung 60 kann intrakorporal oder extrakorporal verwendet werden, wobei bei einer intrakorporalen Verwendung der Steckverbindung 60 nur eine kleine Anzahl perkutaner Drähte vorgesehen sein muss. Die Infektionsgefahr kann hierdurch erheblich verringert werden. Die Anordnung der erfindungsgemäßen Schaltung in einer Steckverbindung 60 hat den Vorteil, dass der Stecker unabhängig vom Elektrodenarray an die Erfordernisse eines Ableitungssystems bzw. einer Stimulationseinrichtung angepasst werden kann, ohne das Elektrodenarray selbst anpassen zu müssen.

**Fig. 3** zeigt eine implantierbare Elektrodenanordnung 10 mit einer Anzahl von Elektroden 20, wobei die Schaltung zur Auswahl einer Anzahl von Elektroden 20 der implantierbaren Elektrodenanordnung 10 auf dem Elektrodenträger 30 angeordnet ist. Bei der in Fig. 3 gezeigten Ausführungsform muss lediglich ein perkutanes Kabel 80 vorgesehen sein, in welchem die elektrisch leitfähigen Drähte zum Verbinden der Ausgänge der Signalschnittstelle mit einer Ableitungseinrichtung bzw. einer Stimulationseinrichtung angeordnet sind.

Das perkutane Kabel 80 kann Anschlusskontakte 50 aufweisen zum Anschließen des Ableitsystems bzw. der Stimulationseinrichtung an die elektrisch leitfähigen Drähte des perkutanen Kabels 80. Weil letztlich nur ein perkutanes Kabel 80 vorgesehen ist, kann die Infektionsgefahr erheblich verringert werden. Das Anschließen eines Ableitungssystems bzw. einer Stimulationseinrichtung an das Elektrodenarray wird zudem vereinfacht, was die Betriebssicherheit erhöht, da die Gefahr eines fehlerhaften Anschlusses an die Stimulationseinrichtung bzw. an die Ableitungseinrichtung reduziert wird. Weil die Anzahl perkutaner Kabel gering gehalten werden kann, kann auch die Unfallgefahr, etwa in Operationssälen, verringert werden.

**Fig. 4** zeigt eine mögliche Ausgestaltung einer erfindungsgemäßen Schaltung zur Auswahl einer Anzahl von Elektroden 20 einer implantierbaren Elektrodenanordnung 10. Die Schaltung umfasst im Wesentlichen eine Spule 110, eine Datenextraktionseinrichtung 120, eine Energieerzeugungseinrichtung 130 und eine Schaltelektronik 100.

Die Schaltung wird induktiv über die Spule 110 mit Energie und Daten versorgt, wobei die Daten ein Aktivierungsschema der einzustellenden Schaltzustände der Schalter in der Schaltelektronik 100 umfassen. Die Programmierung der Schaltung bzw. die Versorgung der Schaltung mit Energie erfolgt über eine hier nicht gezeigte Programmiereinheit, welche in die Nähe der Spule 110 gebracht wird, so dass die Programmiereinheit induktiv mit der Spule 110 gekoppelt ist. Die Trägerwelle der induktiven Koppelung wird zur Energieversorgung genutzt, wobei eine Energieerzeugungseinrichtung 130 aus der Trägerwelle eine stabile Versorgungsspannung erzeugt und diese der Schaltelektronik 100 zuführt. Die Daten für die Programmierung der Schaltelektronik werden mit Hilfe der Datenextraktionseinrichtung 120 aus dem Sendesignal, welches vorzugsweise auf die Trägerwelle aufmoduliert ist, extrahiert. Die extrahierten Daten, welche das Aktivierungsschema umfassen, werden der Schaltelektronik 100 zugeführt.

Die Schaltelektronik 100 weist n Eingänge und m Ausgänge auf, wobei die Anzahl der Eingänge größer ist als die Anzahl der Ausgänge. Erfindungsgemäß schaltet die Schaltelektronik eine Anzahl der n Eingänge zu den m Ausgängen durch, wobei das Durchschalten der Eingänge zu den Ausgängen gemäß dem übertragenen Aktivierungsschema erfolgt. Erfindungsgemäß werden die n Eingänge mit den Elektroden 20 der implantierbaren Elektrodenanordnung 10 verbunden. Die m Ausgänge können mit einem Ableitungssystem und/oder mit einem Pulsgenerator verbunden werden.

Nachdem die Eingänge gemäß dem Aktivierungsschema mit den Ausgängen verbunden worden sind, kann die externe Programmiereinheit abgeschaltet und von der Spule 110 entfernt werden. Erfindungsgemäß bleiben nach dem Entfernen der Programmiereinheit von der Spule 110, wenn die Schaltung also nicht mehr mit Energie versorgt wird, die Schaltzustände der Schalter in der Schaltelektronik 110 erhalten. Das bedeutet, dass nach dem Unterbrechen der Energiezufuhr die gewählten Verbindungen zwischen den Eingängen und den Ausgängen erhalten bleiben und so eine Signalübertragung zwischen den Eingängen und den damit verbundenen Ausgängen weiterhin möglich ist.

Nach dem Einstellen der Schaltzustände der Schalter gemäß dem Aktivierungsschema befinden sich sowohl die Elektrodenanordnung als auch die Schaltung in einem passiven Zustand, wobei für den bestimmungsgemäßen Betrieb der Elektrodenanordnung keine Energieversorgung notwendig ist. Eine Energieversorgung wird nur für das Einstellen der Schaltzustände benötigt. Nach dem Einstellen der Schaltzustände kann die Elektrodenanordnung wie ein herkömmliches Elektrodenarray verwendet werden.

Die Schalter der Schaltelektronik 100 können beispielsweise durch feinwerktechnische oder mikrosystemtechnische, bistabile Relais realisiert werden.

Als besonders geeignet haben sich aber elektronische Schaltelemente, etwa MOS-Transistoren mit Ladungsspeicherung über dem Gate erwiesen, um einen dauerhaften Schaltzustand des elektronischen Schaltelementes sicherzustellen.

Diese Schaltelemente ermöglichen ohne dauerhaft angelegten Strom/Spannung bei geringer Schaltenergie/-spannung eine dauerhafte Elektrodenauswahl, ohne beim Umschalten dann Schaltartefakte hervorzurufen, die eine Nervenerregung hervorrufen könnten. Gerade die MOS-Transistoren mit Ladungsspeicherung lassen sich gut in Standard-Elektronik integrieren, benötigen nur wenig Energie, die durch die drahtlose Energieversorgung gut zu realisieren ist.

Mit dieser rein elektronischen Lösung werden mechanische Versagensfälle umgangen. Man kann im Niederspannungsbereich bleiben, wodurch keine hohen Spannungen im Schalter oder der Elektrode notwendig sind. Die Lösung kann vollständig mit Standardtechnologien generiert werden und benötigt weniger "Teile" als eine Lösung z.B. mit Relais. Weiterer Vorzug ist die Artefaktunterdrückung. Relais kann man nur "an-" oder "aus-" schalten, die Schaltgeschwindigkeit ist nicht einstellbar. Dadurch kann es zur elektrischen Stimulation von Nervenzellen kommen. Durch "langsames" Schalten, wie es mit den MOS-Trnsistoren möglich ist, werden diese Schalt-Artefakte unterdrückt.

Mit der vorliegenden Erfindung können beliebig viele Elektroden parallel geschaltet und so die Anzahl und damit die Fläche (also das Hirnareal), über die abgeleitet wird, variiert werden. Dadurch können kleinere oder größere Areale wahlweise abgeleitet / aufgenommen werden.

Zusammenfassend wird also die mögliche Reduktion von Artefakten kombiniert mit einer willkürlichen Auswahl und Kombination von Parallelschaltungen erzielt. Elektroden, die nicht benötigt werden, können weiterhin einzeln "floaten" (also) nicht verbunden sein, oder gemeinsam mit allen anderen nicht benötigten Elektroden zusammen oder in Gruppen zusammen geschaltet werden, die dann "in der Luft hängen" (floating) oder auf eine definiertes Potential gelegt werden.

Es lassen sich aber auch MEM-Resistoren als Schalter der Schaltelektronik 100 verwenden.

In der Datenextraktionseinrichtung 120 werden die mit der Trägerwelle übertragenen Daten aus der Trägerwelle dekodiert und über eine Logik, etwa ein Mikro Controller oder ein Field Programable Gate Array (FPGA), an die Schaltelektronik weitergeleitet. Diese Logik generiert Schaltsignale, die die Schaltelemente in der Schaltelektronik 100 ansteuern und Eingänge mit Ausgängen verbinden oder bereits vorliegende Verbindungen reversibel lösen kann.

Das Einstellen der Schaltzustände über eine drahtlose Programmierschnittstelle, etwa mittels einer induktiv mit der Schaltung gekoppelten Programmiereinheit, erlaubt es, bestimmte Elektrodenkanäle auszuwählen und die Auswahl zeitlich dauerhaft bis zur nächsten Auswahl von Elektrodenkanälen beizubehalten.

Bei der drahtgebundenen Datenübertragung zwischen Elektroden einerseits und dem Ableitsystem bzw. dem Pulsgenerator andererseits wird die maximale Datenrate der Signalübertragung nur durch die physikalischen Eigenschaften der Verbindungskabel beschränkt, welche weit oberhalb der Frequenzbereiche der neuronalen Signale liegt. Dadurch ergeben sich im Vergleich zur drahtlosen Signalübertragung erhebliche Vorteile, weil insbesondere bei Systemen mit vielen Übertragungskanälen die maximale Datenrate bei einer drahtlosen Signalübertragung durch die Trägerfrequenz bzw. das Übertragungssignal beschränkt ist.

Erfindungsgemäß erfolgt also die Signalübertragung zwischen Elektroden und Ableitelektronik bzw. Pulsgenerator kabelgebunden, während die Programmierung der Schaltelektronik drahtlos erfolgt. Dadurch kann der bestimmungsgemäße Betrieb der Elektrodenanordnung ohne eigene Energiezufuhr bewerkstelligt werden. Gleichzeitig kann eine hohe Datenrate für die Signalübertragung gewährleistet werden.

Ein Elektrodenarray, welches mit einer erfindungsgemäßen Schaltung zur Auswahl einer Anzahl von Elektroden gekoppelt ist, hat zudem den Vorteil, dass die konkrete Auswahl von Elektroden erst dann erfolgen muss, wenn die Elektrodenanordnung etwa in einem Sulcus eines Gehirns eingesetzt worden ist.

Bei der Epilepsie-Diagnostik oder bei der Diagnostik anderer Erkrankungen, bei denen bioelektrische Signale von einer Gehirnoberfläche abgeleitet werden müssen, ist der Ort, an dem für die jeweilige Anwendung die bioelektrischen Signale auftreten, nur näherungsweise bekannt. Bedingt durch die Plastizität des Gehirns kann sich der Ort, an dem die jeweiligen bioelektrischen Signale auftreten über die Implantationszeit hinweg ändern. Zudem ist eine exakte Platzierung der Elektrodenanordnung bzw. der Elektroden während einer Operation in vielen Fällen nicht hinreichend möglich.

Erfindungsgemäß können nach der Implantation der Elektrodenanordnung sämtliche Elektrodenkanäle begutachtet werden und anschließend nur noch jene Elektrodenkanäle ausgewählt werden, welche für die jeweilige Anwendung relevant sind. Die Auswahl der relevanten Elektrodenkanäle erfolgt, wie vorstehend beschrieben, durch Einstellen der Schaltzustände der entsprechenden Schalter. Ändert sich aufgrund der Plastizität des Gehirns die räumliche Lage der Elektroden relativ zu dem Gehirn, so kann die Elektrodenanordnung mit Hilfe eines angepassten Aktivierungsschemas an die sich geänderten Umstände angepasst werden. Eine Energieversorgung der Elektrodenanordnung bzw. der erfindungsgemäßen Schaltung ist dabei nur für das Anpassen der Schaltzustände an die geänderten Bedingungen erforderlich.

Das erfindungsgemäße Verfahren zur Herstellung einer Elektrodenanordnung mit einer Vielzahl von Elektroden umfasst die folgenden Schritte:
- Anschließen der Vielzahl von Elektroden an die Elektrodenanschlüsse einer Signalschnittstelle einer erfindungsgemäßen Schaltung,
- nach dem Anschließen der Elektroden an die Elektrodenanschlüsse, verbinden einer Anzahl von Elektroden mit den Signalanschlüssen der Signalschnittstelle, wobei das Verbinden folgende Schritte umfasst
   - Versorgen der Schaltelektronik der Auswahlschaltung mit Energie über die Programmierschnittstelle der Auswahlschaltung,
   - Übertragen von Schaltsignalen an die Schaltelektronik der Auswahlschaltung über die Programmierschnittstelle der Auswahlschaltung, wobei die Schaltsignale einzustellende Schaltzustände der Schalter der Schalterelektronik umfassen, und
   - Beenden des Versorgens der Schaltelektronik mit Energie und der Übertragung von Schaltsignalen, sobald die eingestellten Schaltsignale der Schalter den einzustellenden Schaltzuständen entsprechen.

Vorzugsweise kann langsames Schalten bewirkt werden, um Schalt-Artefakte zu vermeiden.

Dies ermöglicht eine besonders einfache und effiziente Herstellung der Elektrodenanordnung, d.h. das schnelle Anschweißen der Anschlusskabel an die Elektrode in beliebiger Auswahl - um erst nach dem Anschweißen über die programmierbare Schnittstelle eine Zuordnung der Elektroden an die Ausgänge durchzuführen, vorzugsweise in einer Standardkonfiguration.

Vorteilhaft ist es, das Verbinden der Anzahl von Elektroden mit den Signalanschlüssen der Signalschnittstelle durchzuführen, nachdem ein Aktivierungsschema für die Elektroden bestimmt worden ist. Vorteilhaft ist hierbei, dass die Schaltzustände der Schalter erst dann festgelegt werden müssen, wenn der konkrete Einsatz der Elektrodenanordnung feststeht. Besonders vorteilhaft ist es, dass die Schaltzustände der Schalter erst dann festgelegt werden müssen, nachdem die Elektrodenanordnung in einen Sulcus oder an der Gehirnoberfläche eingesetzt worden ist.

Somit erübrigt sich eine Kennzeichnung der Anschlusskabel vor dem Einsetzen der Elektrodenkonfiguration. Denn die Kabel können nach dem Einsetzen dann den Elektroden in beliebiger Weise durch entsprechende Programmierung der Schaltzustände der Schalter zugeordnet werden.

## Patentansprüche

1. Vorrichtung, umfassend eine Auswahlschaltung und eine implantierbare Elektrodenanordnung mit Elektroden (20) und einem Elektrodenträger (30) zur Auswahl einer Anzahl der Elektroden (20) der implantierbaren Elektrodenanordnung (10), wobei die Auswahlschaltung umfasst:
- eine Signalschnittstelle mit einer Anzahl von Elektrodenanschlüssen (21), welche mit der Anzahl von Elektroden (20) der Elektrodenanordnung (10) gekoppelt sind, und einer Anzahl von Signalanschlüssen (22), wobei die Signalschnittstelle zur drahtgebunden Signalübertragung zwischen den Elektroden und den Signalanschlüssen ausgestaltet ist,
- eine Schaltelektronik (100) mit einer Anzahl von programmierbaren Schaltern, wobei ein Schalter in einem Schaltzustand einen Elektrodenanschluss (21) mit einem Signalanschluss (22) verbindet und in einem zweiten Schaltzustand den Elektrodenanschluss von dem Signalanschluss (22) trennt, wobei das Versorgen der Schaltelektronik (100) mit Energie drahtlos erfolgt,
- eine Programmierschnittstelle, über welche die Schaltzustände der Schalter gemäß einem Aktivierungsschema für die Elektroden (20) einstellbar sind und über welche die Schaltelektronik (100) für das Einstellen der Schaltzustände der Schalter mit Energie versorgbar ist, wobei die Schalter ausgestaltet sind, ihre über die Programmierschnittstelle eingestellten Schaltzustände beizubehalten, wenn die Energiezufuhr unterbrochen wird,
die Schalter als MOS-Transistoren mit Ladungsspeicherung ausgestaltet sind,
und wobei
die Programmierschnittstelle zur drahtlosen Übertragung des Aktivierungsschemas an die Schaltelektronik ausgestaltet ist,
die Programmierschnittstelle als induktive Programmierschnittstelle ausgestaltet ist und induktiv mit einer Programmiereinheit koppelbar ist und die Programmierschnittstelle eine Einrichtung (130) zum Erzeugen einer Versorgungsspannung für die Schaltelektronik (100) aus einer Trägerwelle der induktiven Koppelung aufweist und
die Elektroden (20) der Elektrodenanordnung (10) und die Auswahlschaltung auf dem Elektrodenträger (30) angeordnet sind.

2. Vorrichtung nach Anspruch 1,wobei die Anzahl von Signalanschlüssen ungleich der Anzahl von Elektrodenanschlüssen ist, insbesondere kleiner als die Anzahl von Elektrodenanschlüssen.

3. Vorrichtung nach einem der vorherigen Ansprüche, wobei die Programmierschnittstelle eine Einrichtung (120) zum Extrahieren von Daten aus der Trägerwelle der induktiven Koppelung aufweist, wobei die extrahierten Daten einzustellende Schaltzustände der Schalter der Schaltelektronik (100) umfassen.

4. Verfahren zum Betrieb einer Vorrichtung nach einem der vorhergehenden Ansprüche gemäß einem Aktivierungsschema, welches die einzustellenden Schaltzustände der Schalter der Schaltelektronik (100) umfasst, umfassend:
- drahtloses Versorgen der Schaltelektronik (100) mit Energie über die Programmierschnittstelle der Auswahlschaltung,
- drahtloses Übertragen von Schaltsignalen an die Schaltelektronik (100) über die Programmierschnittstelle der Auswahlschaltung, wobei die Schaltsignale das Aktivierungsschema umfassen,
- Beenden der Energieversorgung der Schaltelektronik und der Übertragung von Schaltsignalen, sobald die eingestellten Schaltzustände der Schalter dem Aktivierungsschema entsprechen; und
- drahtgebundene Signalübertragung zwischen den Signalanschlüssen und den gemäß dem Aktivierungsschema mit den Signalanschlüssen (22) verbundenen Elektroden (20).

5. Verfahren nach Anspruch 4, wobei die Elektrodenanordnung (10) in einem Teil des zentralen oder peripheren Nervensystems einen Kontakt mit dem Teil herstellt und insbesondere in einem Sulcus eines Gehirns eingesetzt ist.

## Claims

1. A device comprising a selection circuit and an implantable electrode arrangement including electrodes (20) and an electrode support (30), permitting to select a number of the electrodes (20) of the implantable electrode arrangement (10), said selection circuit comprising:
- a signal interface having a number of electrode connections (21) which are coupled to the number of electrodes (20) of said electrode arrangement (10), and a number of signal connections (22), wherein
said signal interface is arranged for wired signal transmission between the electrodes and the signal connections,
- a switching electronics (100) having a number of programmable switches, wherein a switch, when it is in one connection state, connects an electrode connection (21) with a signal connection (22) and, when it is in a second switching state, disconnects the electrode connection from the signal connection (22), wherein
the supply of the switching electronics (100) with energy takes place in a wireless manner,
- a programming interface via which the switching states of the switches may be set for the electrodes (20) in accordance with an activation scheme and via which the switching electronics (100) is capable of being supplied with energy for setting the switching states of the switches, wherein the switches are configured to maintain the switching states set via the programming interface when the energy supply is interrupted,
said switches being configured as MOS transistors with charge storage,
and wherein
the programming interface is configured for wireless transmission of the activation scheme to the switching electronics,
the programming interface is configured as an inductive programming interface and may be inductively coupled to a programming unit, and the programming interface has a means (130) for creating a supply voltage for the switching electronics (100) from a carrier wave of the inductive coupling, and
the electrodes (20) of the electrode arrangement (10) and the selection circuit are disposed on the electrode support (30).

2. The device as claimed in claim 1, wherein the number of signal connections is not equal to the number of electrode connections and is, in particular, smaller than the number of electrode connections.

3. The device as claimed in any of the preceding claims, wherein the programming interface has a means (120) for extracting data from the carrier wave of the inductive coupling, the extracted data comprising switching states to be set for the switches of the switching electronics (100).

4. A method for operating a device as claimed in any of the preceding claims in accordance with an activation scheme which comprises the switching states to be set for the switches of the switching electronics (100), said method comprising:
- wirelessly supplying the switching electronics (100) with energy via the programming interface of the selection circuit,
- wirelessly transmitting switching signals to the switching electronics (100) via the programming interface of the selection circuit, said switching signals comprising the activation scheme,
- terminating the energy supply of the switching electronics and the transmission of switching signals once the set switching states of the switches correspond to the activation scheme; and
- wiredly transmitting signals according to the activation scheme between the signal connections and the electrodes (20) that are connected to the signal connections (22).

5. The method as claimed in claim 4, wherein the electrode arrangement (10) establishes contact in a portion of the central or peripheral nervous system with said portion and is employed, in particular, in a sulcus of a brain.

## Revendications

1. Dispositif, lequel comprend un circuit sélecteur et une disposition d'électrodes implantable présentant des électrodes (20) et un support des électrodes (30), en vue de sélectionner un nombre donné des électrodes (20) de la disposition d'électrodes implantable (10), le circuit sélecteur comprenant :
- une interface de signaux avec un nombre donné de connexions d'électrodes (21) couplées au nombre d'électrodes (20) de la disposition d'électrodes (10), et avec un nombre donné de connexions de signaux (22),
l'interface de signaux étant conçue pour permettre la transmission de signaux par fil entre les électrodes et les connexions de signaux,
- une électronique de commutation (100) avec un nombre donné de commutateurs programmables, un commutateur reliant, dans un état de commutation donné, une connexion d'électrodes (21) à une connexion de signaux (22) et coupant, dans un deuxième état de commutation, la connexion d'électrodes de la connexion de signaux (22),
l'alimentation en énergie de l'électronique de commutation (100) s'effectuant sans fil,
- une interface de programmation par le biais de laquelle les états de commutation des commutateurs peuvent être réglés conformément à un schéma d'activation prévu pour les électrodes (20) et par le biais de laquelle l'électronique de commutation (100) peut être alimentée en énergie en vue du réglage des états de commutation des commutateurs, les commutateurs étant conçus pour garder leurs états de commutation réglés par le biais de l'interface de programmation lorsque l'alimentation en énergie est interrompue, les commutateurs étant conçus en tant que transistors MOS avec stockage de charge,
et
l'interface de programmation étant conçue pour transmettre sans fil le schéma d'activation à l'électronique de commutation, l'interface de programmation étant conçue en tant qu'interface de programmation inductive et pouvant être couplée de manière inductive à une unité de programmation, et l'interface de programmation présentant un organe (130) destiné à générer une tension d'alimentation pour l'électronique de commutation (100) à partir d'une onde porteuse du couplage inductif, et
les électrodes (20) du dispositif d'électrodes (10) et le circuit sélecteur étant disposés sur le support d'électrodes (30).

2. Dispositif selon la revendication 1, dans lequel le nombre de connexions de signaux n'est pas identique au nombre de connexions d'électrodes, en particulier est inférieur au nombre de connexions d'électrodes.

3. Dispositif selon l'une quelconque des revendications précédentes, dans lequel l'interface de programmation présente un organe (120) destiné à extraire des données de l'onde porteuse du couplage inductif, les données extraites comprenant des états de commutation des commutateurs de l'électronique de commutation (100) qui doivent être réglés.

4. Procédé destiné à faire fonctionner un dispositif selon l'une quelconque des revendications précédentes conformément à un schéma d'activation qui comprend les états de commutation des commutateurs de l'électronique de commutation (100) qui doivent être réglés, lequel procédé comprenant :
- l'alimentation en énergie sans fil de l'électronique de commutation (100) par le biais de l'interface de programmation du circuit sélecteur,
- la transmission de signaux de commutation sans fil à l'électronique de commutation (100) par le biais de l'interface de programmation du circuit sélecteur, les signaux de commutation comprenant le schéma d'activation,
- l'arrêt de l'alimentation en énergie de l'électronique de commutation et de la transmission de signaux de commutation dès que les états de commutation des commutateurs correspondent au schéma d'activation ; et
- la transmission de signaux par fil entre les connexions de signaux et les électrodes (20) reliées auxdites connexions de signaux (22) selon le schéma d'activation.

5. Procédé selon la revendication 4, lors duquel la disposition d'électrodes (10) placée dans une partie du système nerveux central ou périphérique établit un contact avec ladite partie et est en particulier utilisée dans un sulcus d'un cerveau.
